# EUROPEAN PATENT APPLICATION

(11) **EP 0 552 551 A1**
(43) Date of publication of application: **28.07.1993**
(21) Application number: 92311422.7
(22) Date of filing: 15.12.1992
(51) Int. Cl.: C07H 15/14

(54) **Preparation of thioglycosides**

(30) Priority: 17.01.1992 GB 9201000
(71) Applicant: IMPERIAL CHEMICAL INDUSTRIES PLC, London SW1P 3JF (GB)
(72) Inventor: Rennie, Robert Allan Campbell, Appleton, Warrington (GB); Johnson, Trevor, Frodsham, Cheshire (GB)
(74) Representative: Roberts, Jonathan Winstanley

(57) **Abstract**

A process for the preparation of a thioglycoside of a saccharide which process comprises the step of reacting the saccharide, eg glucose, with a thiol, eg an aliphatic thiol, in the presence of an aqueous halocarboxylic or halosulphonic acid.

## Description

The present invention relates to a process for the preparation of thioglycosides of saccharides, particularly of monosaccharides, more particularly of glucose.

Thioglycosides of monosaccharides and the preparation thereof are known. Typically, they are prepared by a multistage process involving protection and activation of the monosaccharide prior to reaction with a thiol. In an alternative process the reaction of a saccharide with a thiol in the presence of liquid hydrogen fluoride or pyridinium poly hydrogen fluoride has been suggested.

We have now invented a process for the preparation of thioglycosides directly from saccharides which overcomes the disadvantages of the aforementioned processes.

According to the present invention there is provided a process for the preparation of a thioglycoside of a saccharide which process comprises the step of reacting the saccharide with a thiol in the presence of an aqueous halocarboxylic acid or halosulphonic acid.

The saccharide used in the process according to the present invention is preferably a monosaccharide, although we do not exclude the possibility that it may be a disaccharide, eg maltose, lactose, or an oligosaccharide, eg dextrin.

As examples of monosaccharides which may be used in the process according to the present invention may be mentioned inter-alia galactose, mannose, xylose, arabinose or preferably glucose.

As examples of suitable thiols which may be used in the process according to the present invention may be mentioned inter alia hydrocarbyl thiols, e.g. aromatic thiols or preferably aliphatic thiols, more preferably C₄-C₁₈ thiols. We do not exclude the possibility that the thiol may bear one or more substituents which do not impede unduly the process of the present invention. For example, a hetero-atom may be present in the thiol, eg HSCH₂CH₂OCH₂CH₃ or HO(CH₂)ₙSH.Furthermore, a dithiol may be used in the process of the present invention.

As examples of halocarboxylic acids and halosulphonic acids for use in the process of the present invention (which acids are hereinafter referred to for convenience as "strong acids") may be mentioned inter alia fluorosulphonic acids, perfluorosulphonic acids or preferably a fluorocarboxylic acid, more preferably trifluoroacetic acid.

The process according to the present invention may be carried out in acid/water mixtures wherein the strong acid provides the major component of the mixture by volume, i.e. more than 50% v/v, or in neat acid. Preferably it is carried out in an acid/water mixture comprising between 70:30 by volume acid: water to 90:10 by volume acid: water.

Typically, the process according to the present invention is carried out at between 40°C and 80°C , preferably at about 75°C, for between 1-18 hours.

Depending on the particular saccharide and thiol used in the process according to the present invention, the skilled man will be able to find by simple experiment suitable reaction conditions, e.g. acid concentration, temperature and time, for carrying it out.

The thioglycoside product can be readily isolated from the reaction mixture by evaporation of the strong acid, preferably under reduced pressure or as an azeotrope with water. Alternatively, it may be isolated by solvent extraction to afford an aqueous solution or dispersion, from which the anhydrous product may be obtained by conventional methods.

The present invention is further illustrated by reference to the following Examples.

### Example 1

This Example illustrates the preparation of an alkyl thioglycoside by the process according to the present invention.

Octanethiol (42.05 g, 0.287 mol) was added to a solution of glucose (50.02 g, 0.278 mol) in trifluoroacetic acid (90 cm³) and water (10 cm³) at 75°C under a nitrogen atmosphere, over 1½ hours. The solution was stirred at 70-75°C for 3 hours. The solution was concentrated under reduced pressure at 35°C leaving a syrup which was co-evaporated with methanol (200 cm³). The resulting gum was disolved in methanol (300 cm³) and the solution was neutralised with ammonia solution and treated with activated carbon (5-10 g). The mixture was filtered and the filtrate was concentrated under reduced pressure yielding 71.74 g (83.9%) of a brown syrup. The brown syrup was found to have [α]_{D} + 74.4 (c = 0.95 in methanol) and the same TLC characteristics as an authentic sample of ocytyl thioglucoside, Rf 0.48 (silica plates, 12:3:1 ethyl acetate: methanol: water as eluant). The structure of the product was confirmed by mass spectrometry (electron impact) mass of 331 (parent ion + sodium).

For further structural determination, a sample of the product was acetylated in pyridine using acetic anhydride at 0-5°C for 2 days. The pyridine solution was concentrated under reduced pressure at 35°C leaving an oil which was taken up in chloroform. The chloroform solution was washed with water, dried over magnesium sulphate and filtered and the filtrate was concentrated to an oil. The oil was shown to be a 1:1 mixture of alpha and beta isomers by TLC;Rf 0.51 (silica plates 1:1 hexane: ethyl acetate as eluant). The structure was confirmed as the tetra-acetate derivative by ¹H NMR(CDCl₃,500 MHz), characteristic signals (ppm), 2.0-1.9 (8 singlets, 3 H each)[CH₃-C=0]; 4.42 (doublet, 1H)[βeta anomeric proton], 5.56 (doublet, 1H)[alpha anomeric proton]. It was found to have [α]_{D} + 63.7(C=1.1 in CHCl₃)

## Claims

1. A process for the preparation of a thioglycoside of a saccharide which process comprises the step of reacting the saccharide with a thiol in the presence of an aqueous halocarboxylic or halosulphonic acid.

2. A process as claimed in Claim 1 wherein the saccharide is a monosaccharide.

3. A process as claimed in Claim 2 wherein the monosaccharide is glucose.

4. A process as claimed in Claim 1 wherein the thiol is an aliphatic thiol.

5. A process as claimed in Claim 4 wherein the aliphatic thiol bears 4 to 18 carbon atoms.

6. A process as claimed in Claim 1 wherein the halocarboxylic or halosulphonic acid is a fluorocarboxylic acid.

7. A process as claimed in Claim 6 wherein the fluorocarboxylic acid is trifluoroacetic acid.

8. A process as claimed in Claim 1 wherein the aqueous halocarboxylic or halosulphonic acid is an acid/water mixture comprising between 70:30%v/v and 90:10%v/v acid:water.

9. A process as claimed in Claim 1 carried out at about 75°C.
